# EUROPEAN PATENT APPLICATION

(11) **EP 0 537 980 A1**
(43) Date of publication of application: **21.04.1993**
(21) Application number: 92309308.2
(22) Date of filing: 13.10.1992
(51) Int. Cl.: C07D 295/14, C07D 239/42, C07D 211/34, C07D 213/78, A61K 31/495, A61K 31/505, A61K 31/445, A61K 31/44

(54) **Cyclohexaneacetic acid derivatives as inhibitors of fibrinogen-dependent blood platelet aggregation**

(30) Priority: 16.10.1991 GB 9122016
(71) Applicant: GLAXO GROUP LIMITED, Greenford, Middlesex UB6 0NN (GB)
(72) Inventor: Porter, Barry, Glaxo Group Research Ltd., Hertfordshire SG12 0DP (GB); Eldred, Colin David, Glaxo Group Research Ltd., Hertfordshire SG12 0DP (GB); Kelly, Henry Anderson, Glaxo Group Research Ltd., Hertfordshire SG12 0DP (GB)
(74) Representative: Brewer, Christopher Laurence, Dr.

(57) **Abstract**

The invention relates to cyclohexaneacetic acid derivatives of formula (I) 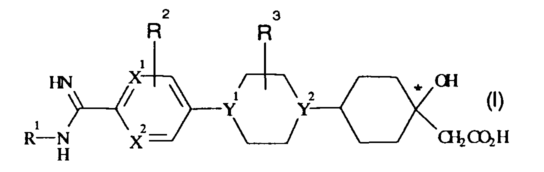
and salts and solvates thereof, in which
X¹, X² and Y¹, which may be the same or different, represent CH or N;
Y² represents N or, when Y¹ represents N, may also represent CH;
R¹ represents a hydrogen atom or a hydroxyl, C₁₋₄alkyl or 2,2,2- trifluoroethyl group;
R² represents a hydrogen atom or, when both X¹ and X² represent CH, may also represents a fluorine, chlorine or bromine atom or a phenyl or C₁₋₄alkyl group;
R³ represents a hydrogen atom or, when both Y¹ and Y² represent N, may also represents a C₁₋₄alkyl or hydroxymethyl group.

The compounds inhibit fibrinogen - dependent blood platelet aggregation.

## Description

This invention relates to cyclohexane derivatives, to processes for their preparation, to pharmaceutical compositions containing such compounds and to their use in medicine.

It is widely accepted that the glycoprotein complex Gp IIb/IIIa is the fibrinogen binding site on platelets that mediates the adhesive function required for platelet aggregation and thrombus formation. We have now found a group of non-peptidic compounds which inhibit fibrinogen-dependent platelet aggregation by blocking the binding of fibrinogen to the putative fibrinogen receptor Gp IIb/IIIa complex.

The invention thus provides the compounds of formula (I) 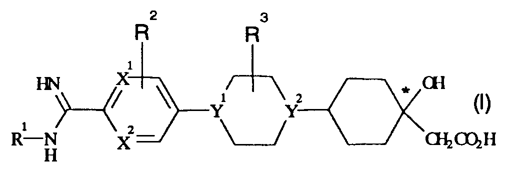
and salts and solvates thereof, in which
X¹, X² and Y¹, which may be the same or different, represent CH or N;
Y² represents N or, when Y¹ represents N, may also represent CH;
R¹ represents a hydrogen atom or a hydroxyl, C₁₋₄alkyl or 2,2,2-trifluoroethyl group;
R² represents a hydrogen atom or, when both X¹ and X² represent CH, may also represents a fluorine, chlorine or bromine atom or a phenyl or C₁₋₄alkyl group;
R³ represents a hydrogen atom or, when both Y¹ and Y² represent N, may also represents a C₁₋₄alkyl or hydroxymethyl group.

In the formulae that follow, the terms "ring -A-" and "ring -B-" will hereinafter be used to described certain rings of formula (I):

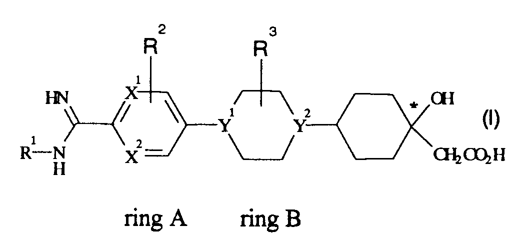

It will be appreciated that, for pharmaceutical use, the salts referred to above will be the physiologically acceptable salts, but other salts may find use, for example in the preparation of compounds of formula (I) and the physiologically acceptable salts thereof.

It will be further appreciated by those skilled in the art that the compounds of formula (I) contain at least one chiral centre (shown as * in formula (I)) and that such compounds exist in the form of a pair of optical isomers (i.e. enantiomers). The invention includes all such isomers and mixtures thereof including racemic mixtures.

Suitable physiologically acceptable salts of the compounds of formula (I) include acid addition salts formed with inorganic or organic acids (for example hydrochlorides, hydrobromides, sulphates, phosphates, benzoates, naphthoates, hydroxynaphthoates, p-toluenesulphonates, methanesulphonates, sulphamates, ascorbates, tartrates, salicylates, succinates, lactates, glutarates, glutaconates, acetates, tricarballylates, citrates, fumarates and maleates) and inorganic base salts such as alkali metal salts (for example sodium salts).

Other salts of the compounds of formula (I) include salts formed with trifluoroacetic acid.

It is to be understood that the present invention encompasses all isomers of the compounds of formula (I) and their salts and solvates, including all tautomeric and optical forms, and mixtures thereof (e.g. racemic mixtures).

The term 'alkyl' as a group or part of a group means a straight or branched chain alkyl group, for example a methyl, ethyl, n- propyl, i-propyl, n-butyl, s-butyl or t-butyl group.

A preferred group of compounds of the invention are those of formula (Ia) 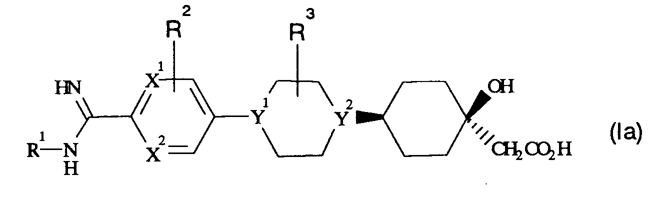
and salts and solvates thereof, in which X¹, X², Y¹, Y², R¹, R² and R³ are as defined in formula (I) above.

A particularly preferred compound of the invention is cis-4-[4- [4-(aminoiminomethyl)phenyl]-1-piperazinyl]-1- hydroxycyclohexaneacetic acid and physiologically acceptable salts and solvates thereof.

Other preferred compounds of the invention are
cis-4-[4-[4-(aminoiminomethyl)-2-bromophenyl]-1-piperazinyl]-1-hydroxycyclohexaneacetic acid;
cis-4-[4-[2-(Aminoiminomethyl)-5-pyrimidinyl]-1-piperazinyl]-1-hydroxycyclohexaneacetic acid;
cis-4-[4-[2- (aminoiminomethyl)-5-pyridinyl]-1-piperazinyl]-1-hydroxycyclohexaneacetic acid ;
cis-4-[4-[4-(aminoiminomethyl)phenyl]-1-piperidinyl]-1-hydroxycyclohexaneacetic acid;
cis-4-[4-[5-(aminoiminomethyl)[1,1′-biphenyl]-2-yl]-1-piperazinyl]-hydroxycyclohexaneacetic acid;
and physiologically acceptable salts and solvates thereof.

Compounds of formula (I) inhibit blood platelet aggregation as demonstrated by studies performed on human gel filtered platelets (GFP) using a Born-type optical aggregometer (Born, G.V., 1962, Nature, 194, 927-929).

In view of their fibrinogen antagonist activity, the compounds of the present invention are of interest for use in human and veterinary medicine, particularly in the treatment or prophylaxis of thrombotic disorders. Particular examples of thrombotic disorders are known in the art and include occlusive vascular diseases such as myocardial infarction, cardiac fatalities, angina, transient ischaemic attacks and thrombotic stroke, arteriosclerosis, vessel wall disease, peripheral vascular disease, nephropathy, retinopathy, postoperative thrombosis, pulmonary embolism, deep vein thrombosis and retinal vein thrombosis. The compounds of the invention are also of interest for use in the prophylaxis of peri- and postoperative complications following organ transplantation (particularly cardiac and renal), coronary artery bypass, peripheral artery bypass, angioplasty, thrombolysis and endarterectomy.

The compounds of the invention may also be useful for the treatment or prophylaxis of other conditions in which the glycoprotein complex Gp IIb/IIIa or other integrin receptors are implicated. Thus, for example, the compounds of the invention may potentiate wound healing and be useful in the treatment of osteoporosis.

The compounds of the invention may also be useful for the treatment of certain cancerous diseases. For example, compounds of the invention may be of use to prevent or delay metastasis in cancer.

According to a further aspect of the invention, we provide a compound of formula (I) or a physiologically acceptable salt or solvate thereof for use in human or veterinary medicine, particularly for use in the treatment or prophylaxis of thrombotic disorders.

According to another aspect of the invention, we provide the use of a compound of formula (I) or a physiologically acceptable salt or solvate thereof for the manufacture of a therapeutic agent for the treatment or prophylaxis of thrombotic disorders.

According to a further aspect of the invention, we provide a method of treating a human or animal subject suffering from or susceptible to a thrombotic disorder, which method comprises administering to said subject an effective amount of a compound of formula (I) or a physiologically acceptable salt or solvate thereof.

It will be appreciated that the compounds of formula (I) may advantageously be used in conjunction with one or more other therapeutic agents. Examples of suitable agents for adjunctive therapy include thrombolytic agents or any other compound stimulating thrombolysis or fibrinolysis and cytotoxic drugs. It is to be understood that the present invention covers the use of a compound of formula (I) or a physiologically acceptable salt or solvate thereof in combination with one or more other therapeutic agents.

The compounds of formula (I) and their physiologically acceptable salts and solvates are conveniently administered in the form of pharmaceutical compositions. Thus, in another aspect of the invention, we provide a pharmaceutical composition comprising a compound of formula (I) or a physiologically acceptable salt or solvate thereof adapted for use in human or veterinary medicine. Such compositions may conveniently be presented for use in conventional manner in admixture with one or more physiologically acceptable carriers or excipients.

The compounds according to the invention may be formulated for administration in any suitable manner. The compounds may, for example, be formulated for topical administration or administration by inhalation or, more preferably, for oral or parenteral administration.

For oral administration, the pharmaceutical composition may take the form of, for example, tablets, capsules, powders, solutions, syrups or suspensions prepared by conventional means with acceptable excipients.

For parenteral administration, the pharmaceutical composition may be given as an injection or a continuous infusion (e.g. intravenously, intravascularly or subcutaneously). The compositions may take such forms as suspensions, solutions or emulsions in oily or aqueous vehicles and may contain formulatory agents such as suspending, stabilising and/or dispersing agents. For administration by injection these may take the form of a unit dose presentation or as a multidose presentation preferably with an added preservative.

Alternatively for parenteral administration the active ingredient may be in powder form for reconstitution with a suitable vehicle.

The compounds of the invention may also be formulated as a depot preparation. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

As stated above, the compounds of the invention may also be used in combination with other therapeutic agents. The invention thus provides, in a further aspect, a combination comprising a compound of formula (I) or a physiologically acceptable salt or solvate thereof together with another therapeutic agent, in particular a thrombolytic agent.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a physiologically acceptable salt or solvate thereof is used in combination with a second therapeutic agent active against the same disease state the dose of each compound may differ from that when the compound is used alone. Appropriate doses will be readily appreciated by those skilled in the art.

A proposed daily dosage of a compound of formula (I) for the treatment of man is 0.01 mg/kg to 30 mg/kg, which may be conveniently administered in 1 to 4 doses. The precise dose employed will depend on the age and condition of the patient and on the route of administration. Thus, for example, a daily dose of 0.1 mg/kg to 10mg/kg may be suitable for systemic administration.

Suitable methods for the preparation of compounds of formula (I) and salts and solvates thereof are described below.

In the formulae that follow, X¹, X², Y¹, Y², R¹, R² and R³ are as defined in formula (I) above unless otherwise stated; R^{p} represents a protecting group and Hal represents a halogen, e.g.bromine.

Thus, according to a first process (A), compounds of formula (I) in which R¹ represents a hydrogen atom may be prepared from compounds of formula (II) 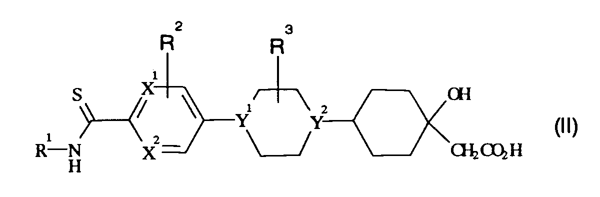
by treating said compounds of formula (II) with a suitable alkylating agent, followed by reaction with a source of ammonia (e.g. ammonium acetate) in an alcoholic solvent (e.g. methanol) at an elevated temperature (e.g. reflux), and thereafter removing the carboxylic acid protecting group. The alkylation (e.g. ethylation) may conveniently be effected by employing an appropriate trialkyloxonium salt (e.g. triethyloxonium tetraflouroborate) in a suitable solvent (e.g. dichloromethane) at room temperature. Alternatively the alkylation (e.g. methylation) may conveniently be effected using an alkyl halide (e.g. iodomethane) in a suitable solvent such as a ketone (e.g. acetone) at an elevated temperature (e.g. reflux).

According to a further process (B), compounds of formula (I) in which R¹ represents a hydroxyl group may be prepared from compounds of formula (III) 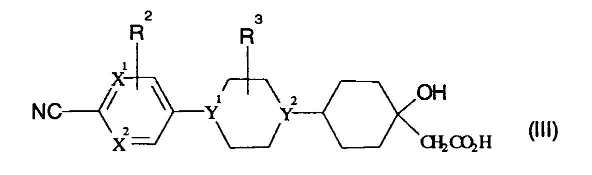
or protected derivatives thereof by treating said compounds of formula (III) with hydroxylamine or an acid addition salt thereof (e.g. hydroxylamine hydrochloride) in the presence of a suitable base such as an alkali or alkaline earth metal carbonate or bicarbonate (e.g. potassium carbonate) and in a solvent such as an alcohol (e.g. methanol), followed, where necessary, by removing any protecting groups present. The reaction with hydroxylamine or an acid addition salt thereof may conveniently be effected at an elevated temperature (e.g. reflux).

According to a yet further process (C), compounds of formula (I) in which R¹ represents a C₁₋₄alkyl or 2,2,2-trifluoroethyl group may be prepared by treating compounds of formula (IV) 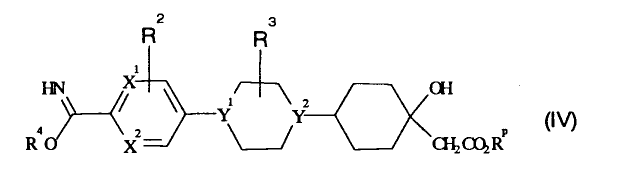
(wherein R⁴ represents an alkyl group, for example a C₁₋₄alkyl group such as ethyl) with an amine R¹NH₂ (where R¹ represents C₁₋₄alkyl or 2,2,2-trifluoroethyl), followed by removal of the carboxylic acid protecting group. The reaction with the amine R¹NH₂ may conveniently be carried out in a suitable solvent such as an alcohol (e.g. methanol) or an ether (e.g. tetrahydrofuran) at about room temperature.

Another process (D) for preparing compounds of formula (I) comprises deprotecting protected derivatives of compounds of formula (I). In a particular embodiment of this process compounds of formula (I) may be prepared from protected carboxyl derivatives of compounds of formula (I).

Suitable carboxyl protection groups include, for example, those described in 'Protective Groups in Organic Synthesis' by Theodora W. Green, second edition, (John Wiley and Sons, 1991) which also describes methods for the removal of such groups. Particular carboxyl protecting groups include, for example, carboxylic acid ester groups such as carboxylic acid alkyl or aralkyl esters, for example where the alkyl or aralkyl portion of the ester function is methyl, ethyl, tert-butyl, methoxymethyl, benzyl, diphenylmethyl, triphenylmethyl or p-nitrobenzyl. When the ester is an unbranched alkyl (e.g. methyl) ester deprotection may be effected under conditions of acid hydrolysis, for example using hydrochloric acid. Tert-butyl and triphenylmethyl ester groups may be removed under conditions of moderate acid hydrolysis, for example using formic or trifluoroacetic acid at room temperature or using hydrochloric acid in acetic acid. Benzyl, diphenylmethyl and nitrobenzyl ester groups may be removed by hydrogenolysis in the presence of a metal catalyst (e.g. palladium).

According to another process (E), compounds of formula (I) may be prepared by interconversion, utilising other compounds of formula (I) as precursors. Thus, for example, compounds of formula (I) in which R¹ represents a hydrogen atom may be prepared from corresponding compounds of formula (I) in which R¹ represents a hydroxyl group by catalytic hydrogenation in a solvent such as an alcohol e.g. ethanol. Suitable catalysts include Raney Nickel or conventional palladium or platinum catalysts.

When a particular isomeric form of a compound of formula (I) is desired, for example a compound of formula (Ia), the required isomer may conveniently be separated using preparative high performance liquid chromatography (h.p.l.c.) applied to the final products of process (A)-(E) above or applied prior to any final deprotection step in said processes.

Compounds of formula (II) may be prepared from compounds of formula (V) 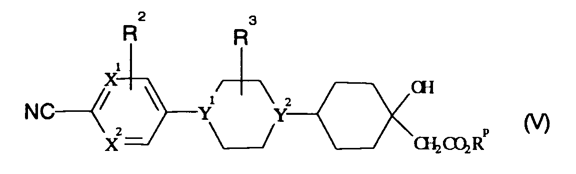
by treating said compounds of formula (V) with hydrogen sulphide. The reaction may conveniently be carried out in a solvent such as dimethylformamide or pyridine and in the presence of an organic base such as an amine (e.g. triethylamine).

Compounds of formula (III) may also be prepared from compounds of formula (V) by removing the carboxylic acid protecting group R^{p} according to the method described in process (D) hereinabove.

Compounds of formula (IV) may also be prepared from compounds of formula (V) by treating said compounds of formula (V) with an alcohol R⁴OH (where R⁴ is as defined previously) under acidic conditions, for example in the presence of an inorganic acid such as hydrochloric acid.

Compounds of formula (V) in which ring -B- represents 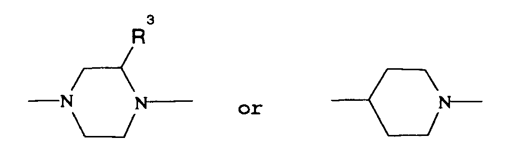
may be prepared by reacting compounds of formula (VI) 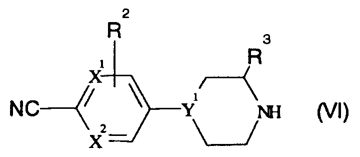
with compounds of formula (VII) 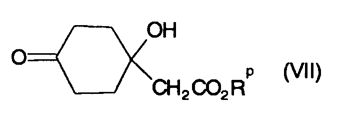
under reducing conditions.

Thus, for example, compounds of formulae (VI) and (VII) may be heated (e.g. at reflux) in an aromatic hydrocarbon solvent (e.g. toluene), preferably containing molecular sieves, and in the presence of an acid such as an alkane- or arylsulphonic acid (e.g. p-toluenesulphonic acid), and the product thereof hydrogenated in an alcoholic solvent (e.g. ethanol) in the presence of a suitable transition metal catalyst such as palladium (e.g. palladium-on- carbon) at about room temperature. Alternatively, compounds of formulae (VI) and (VII) in the presence of an acid such as hydrochloric acid and in a solvent such as an alcohol (e.g. methanol) may be treated with a reducing agent such as a borohydride reducing agent (e.g. sodium cyanoborohydride) and preferably also with molecular sieves.

Compounds of formula (VII) may be prepared from a derivative of cyclohexane-1,4-dione in which one of the two oxo functions is protected (e.g. as a cyclic ketal) by treating said derivative with an anion of an acetic acid derivative CH₃CO₂R^{p} followed by removal of the oxo protecting group. The anion may be generated using an alkyllithium reagent (e.g. n-butyllithium) in an inert solvent such as a hydrocarbon (e.g. n-hexane) or an ether (e.g. tetrahydrofuran) or a mixture of such solvents at a low temperature, optionally in the presence of an organic base such as an amine (e.g. cyclohexylisopropylamine). The oxo protection group may be removed under conventional conditions. Thus, for example, a cyclic ketal may be removed by treatment with a palladium reagent such as bis(acetonitrile)palladium (II) chloride in a solvent such as a ketone (e.g. acetone) at about room temperature or using an acid such as aqueous hydrochloric acid or a mixture of aqueous hydrochloric acid and acetic acid.

Compounds of formula (V) in which ring -B- represents 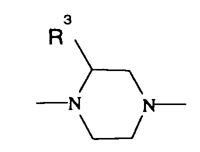
may be prepared by reacting compounds of formula (VIII) 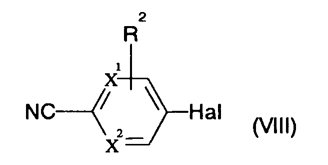
with compounds of formula (IX) 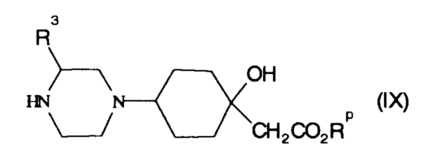
in the presence of a base such as an alkali or alkaline earth metal carbonate or bicarbonate (e.g. sodium bicarbonate) and in a suitable solvent such as an aprotic polar solvent (e.g. dimethylformamide, acetonitrile or dimethylsulphoxide), conveniently at an elevated temperature.

Compounds of formula (IX) may be prepared from compounds of formula (VII) by reacting said compounds with a piperazine derivative or a protected (e.g. N-benzyl protected) piperazine derivative under reducing conditions, for example as described above for the reaction between compounds of formulae (VI) and (VII), followed, where appropriate, by the removal of any N-protecting group present using conventional conditions.

Compounds of formula (V) in which ring -B- represents 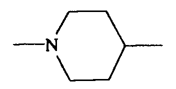
may be prepared from compounds of formula (X) 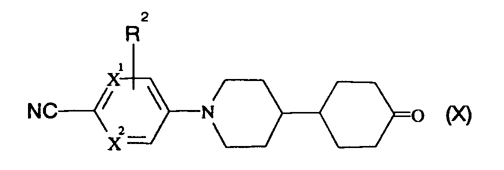
by treating said compounds of formula (X) with an anion of an acetic acid derivative CH₃CO₂R^{p} according to the conditions described above for the preparation of compounds of formula (VII).

Compounds of formula (X) may be prepared by oxidation of compounds of formula (XI) 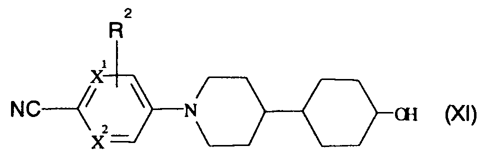
using conventional mild oxidising agents such as pyridinium dichromate in a solvent such as a halogenated hydrocarbon (e.g. dichloromethane).

Compounds of formula (XI) may be prepared by reacting compounds of formula (VIII) with a compound of formula (XII) 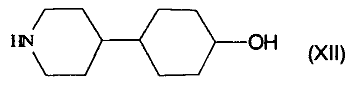
according to the conditions described above for preparing compounds of formula (V) from compounds of formulae (VIII) and (IX).

The compound of formula (XII) may be prepared by hydrogenating a compound of formula (XIII)

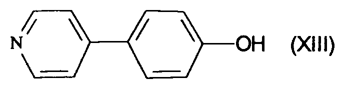

The hydrogenation may conveniently be effected in the presence of a suitable transition metal catalyst such as rhodium, preferably also with alumina, in a solvent such as an alcohol, e.g. ethanol, and preferably at an elevated pressure.

The compound of formula (XIII) is a known compound described by C. Alvarez et al in Synth. Commun. 1991, 21, 619.

Compounds of formula (VI) in which ring -B- represents 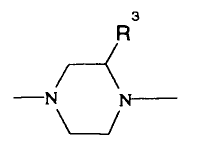
may be prepared by reacting a piperazine 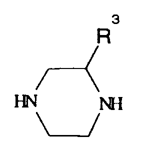
with a compound of formula (VIII) under basic conditions. Suitable bases include alkali or alkaline earth metal carbonates such as sodium carbonate or potassium carbonate. The reaction may conveniently be effected in a solvent such as dimethylformamide or dimethylsulphoxide at an elevated temperature (e.g. 100°-200°C).

Compounds of formula (VI) in which ring -B- represents 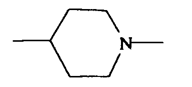
may be prepared from compounds of formula (XIV) 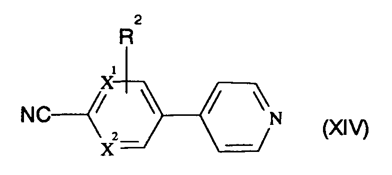
under suitable reducing conditions.

Conveniently, compounds of formula (XIV) are treated with benzyl bromide in an alcoholic solvent (e.g. ethanol) at an elevated temperature to provide a salt of formula (XV) 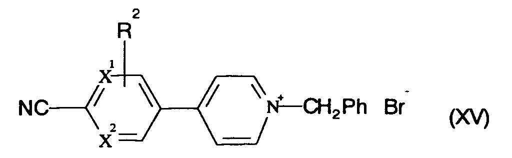
which is reduced, for example using a borohydride reducing agent such as sodium borohydride in a suitable solvent such as an alcohol (e.g. ethanol) or dimethylformamide or a mixture of such solvents to a compound of formula (XVI)

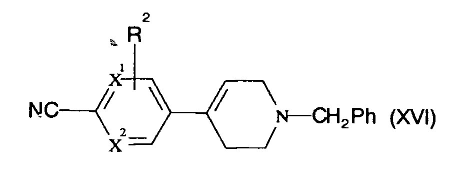

Removal of the benzyl group and double bond from a compound of formula (XVI) provides the desired compounds of formula (VI). The removal of the benzyl group may conveniently be effected by hydrogenolysis in the presence of a palladium catalyst such as Pd(OH)₂-on-carbon, or by reaction with 1-chloroethyl chloroformate in the presence of a base such as 'proton sponge' followed by treatment with methanol. The removal of the double bond may conveniently be effected by hydrogenation in the presence of a platinum catalyst such as platinum-on-carbon or platinum oxide and an acid (e.g. hydrochloric acid).

Compounds of formula (XIV) may be prepared by converting compounds of formula (VIII) to the corresponding boronic acids of formula (XVII) 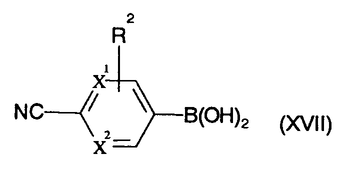
under conventional conditions, and thereafter reacting said compounds of formula (XVII) with a 4-halopyridine such as 4- bromopyridine, preferably in the presence of a suitable transition metal catalyst such as a palladium catalyst [e.g. tetrakis(triphenylphosphine)palladium(0)] and a suitable base such as an alkali metal carbonate (e.g. sodium carbonate). The reaction may conveniently be effected in a solvent such as an aqueous ether (e.g. aqueous 1,2-ethanediol dimethyl ether).

Compounds of formula (XIV) in which ring -A- represents a fluoro, chloro or bromo substituted 1,4-phenylene group may conveniently be prepared by reacting an appropriate compound of formula (VIII) in which Hal is a bromine atom with a compound of formula (XVIII) 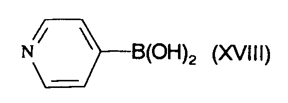
under the boronic acid coupling conditions described previously.

The compound of formula (XVIII) may be prepared from 4- bromopyridine by treating said compound with an alkyllithium reagent (e.g. n-butyllithium) at a low temperature (e.g. about -78°C) in tetrahydrofuran, followed by reaction with a suitable boron reagent such as triisopropyl borate in the presence of an acid (e.g. hydrochloric acid).

Compounds of formula (VIII) are either known compounds or may be prepared from the known compounds of formula (VIII) using conventional chemistry.

Compounds of formula (I) or intermediates thereto in which ring -A- represents a phenyl or C₁₋₄alkyl substituted 1,4-phenylene group may conveniently be prepared by modification of the corresponding compound in which ring -A- represents a bromo substituted 1,4- phenylene group. Thus, for example, the bromo substituted intermediate may be treated with a zinc reagent R²ZnBr (where R² is C₁₋₄alkyl or phenyl) in the presence of a palladium catalyst such as tetrakis (triphenylphosphine)palladium (0). Alternatively, phenylation may be effected using a reagent PhB(OH)₂ in the presence of a palladium catalyst such as tetrakis(triphenylphosphine)palladium (0). Alkylation may also be effected using a tin reagent (R²)₄Sn (where R² is C₁₋₄alkyl) in the presence of a palladium catalyst such as bis (triphenylphosphine)benzylpalladium chloride.

It will also be appreciated by those skilled in the art that of the methods described hereinabove the desired stereochemistry of the product may be obtained either by commencing with an optically pure starting material or by resolving the racemic mixture at any convenient stage in the synthesis.

Resolution of the final product, an intermediate or a starting material may be effected by any suitable method known in the art: see for example 'Stereochemistry of Carbon Compounds' by E L Eliel (McGraw Hill, 1962) and 'Tables of Resolving Agents' by S H Wilen.

Certain intermediates described above are novel compounds, and it is to be understood that all novel intermediates herein form further aspects of the present invention. Compounds of formula (V) are key intermediates and represent a particular aspect of the present invention.

Conveniently, compounds of formula (I) are isolated following work-up as acid addition salts, e.g. trifluoroacetate salts. Physiologically acceptable acid addition salts of the compounds of formula (I) may be prepared from the corresponding trifluoroacetate salts by exchange of ion using conventional means, for example by addition of a suitable organic or inorganic acid. Inorganic base salts of the compounds of formula (I) may also be prepared from the corresponding trifluoroacetate salts by addition of a suitable strong base such as sodium hydride.

Solvates (e.g. hydrates) of a compound of formula (I) may be formed during the work-up procedure of one of the aforementioned process steps.

The following Preparations and Examples illustrate the invention but do not limit the invention in any way. All temperatures are in °C. System (A) is dichloromethane-ethanol-0.880 ammonia. Preparative high performance liquid chromatography (h.p.l.c.) was carried out using a Dynamax 60A C18 8µM 25cm x 41.4mm i.d. column eluted with a mixture of solvents (A) 0.1% trifluoroacetic acid in water and (B) 0.05% trifluoroacetic acid in acetonitrile. Analytical h.p.l.c. was carried out using a Dynamax 60A C18 8µM 25cm x 4.6mm i.d. column using eluants as for preparative h.p.l.c.

### Intermediate 1

### 1,1-Dimethylethyl 8-hydroxy-1,4-dioxaspiro[4.5]decan-8-acetate

n-Butyllithium (1.64M in hexane, 16.1ml) was added to a stirred solution of N-isopropylcyclohexylamine (4.11ml) in dry tetrahydrofuran (50ml) at -76° under nitrogen, and stirring was continued at -76° for 20min. A solution of t-butyl acetate (3.37ml) in tetrahydrofuran (12ml) was added, and stirring was continued at - 76° for 1.25h. A solution of 1,4-dioxaspiro[4,5]decan-8-one (3.02g) in tetrahydrofuran (10ml) was added, and the mixture was allowed to warm to room temperature with stirring overnight. The mixture was partitioned between saturated aqueous ammonium chloride solution (75ml) and ether (3x50ml), and the organic layers were washed with brine, dried (MgSO₄) and evaporated to give a pale yellow oil. Purification by dry flash chromatography on silica gel (Merck 7736) with hexane-ether (4:1-1:1) eluant afforded the title compound (4.23g) as a colourless liquid.
T.l.c. SiO₂ (hexane-ether 1:1), Rf 0.4.

### Intermediate 2

### 1,1-Dimethylethyl 1-hydroxy-4-oxocyclohexaneacetate

Intermediate 1 (0.547g) in acetone (20ml) was treated with bis (acetonitrile)palladium (II) chloride (10mg) and stirred in the dark under nitrogen for 26h. The solvent was removed in vacuo and the residue purified by flash chromatography over silica gel (Merck 60), eluting with hexane-ether (4:1-1:1) and ether, to give the title compound as a colourless solid (0.248g).
T.l.c. SiO₂ (hexane-ether 1:1), Rf 0.2.

### Intermediate 3

### 1,1-Dimethylethyl 1-hydroxy-4-[4-(phenylmethyl)-1-piperazinyl]cyclohexaneacetate

Intermediate 2 (10.0g) and 1-(phenylmethyl)piperazine (9.0g) were dissolved in methanol (250ml) and 1M hydrogen chloride in ethanol (15ml) added, followed by 3 molecular sieves (5g) and sodium cyanoborohydride (2.8g). The mixture was stirred at 22° for 65h, filtered and evaporated in vacuo. The residue was suspended in 8% sodium bicarbonate (100ml) - brine (100ml) and extracted with ethyl acetate (2x250ml). The organic extracts were combined, dried (Na₂SO₄) and evaporated in vacuo. The residual oil was purified by flash chromatography over silica (Merck 9385) with ethertriethylamine (98:2) eluant to afford firstly the trans isomer (2.70g) then a mixture of cis and trans isomers (6.06g) followed by pure cis isomer (1.02g).
T.l.c. SiO₂ (ether-triethylamine 98:2), Rf 0.13 (cis) and 0.18 (trans).

### Intermediate 4

### 1,1-Dimethylethyl 1-hydroxy-4-(1-piperazinyl)cyclohexaneacetate

A 1:1 mixture of cis and trans isomers of Intermediate 3 (6.05g) was dissolved in ethanol (100ml) was treated with 1M ethanolic hydrogen chloride (20ml) and added to pre-hydrogenated Pearlmans catalyst (2.0g) in ethanol (100ml). The mixture was hydrogenated at room temperature and pressure for 16h, filtered through hyflo and evaporated in vacuo. The residue in water (100ml) was treated with 2M sodium carbonate until the pH was 12, then the solution evaporated to dryness in vacuo. The resulting solid was triturated with chloroform (100ml), the residue filtered off and the filtrate evaporated in vacuo to afford the title compound as a colourless viscous gum (4.55g).
T.l.c. SiO₂ (System A 29:10:1), Rf 0.56 (cis) and 0.67 (trans).

### Intermediate 5

### 1,1-Dimethylethyl 4-[4-(2-cyano-5-pyrimidinyl)-1-piperazinyl]-1-hydroxycyclohexaneacetate

5-Bromo-2-cyanopyrimidine¹ (2.62g) and Intermediate 4 (4.47g) in acetonitrile (100ml) were treated with sodium bicarbonate (2.52g), heated at reflux under nitrogen for 22h, cooled and evaporated in vacuo. The residue was partitioned between water (80ml) and ethyl acetate (2x100ml), and the organic extracts dried (Na₂SO₄) and evaporated in vacuo. The residue was purified by flash chromatography over silica (Merck 9385) eluting with dichloromethane-methanol-triethylamine (93:5:2-88:10:2) to afford the title compound (0.890g).
T.l.c. SiO₂ (System A 89:10:1), Rf 0.6.
1. Coll. Czech. Chem. Comm., 1972, 37, 1721.

### Intermediate 6

### 4-(1-Piperazinyl)benzonitrile

4-Chlorobenzonitrile (16.5g) was heated at 180° with piperazine (31g) and sodium carbonate (26.1g) in dimethylsulphoxide (300ml) with stirring under nitrogen for 20h. The mixture was poured into water (1.21) and extracted with chloroform (3x500ml). The organic layers were washed with water, dried (MgS0₄ and evaporated to give the title compound as a yellow oil (23.6g).
T.l.c. Si0₂ (System A 83.5:15:1.5), Rf 0.55.

Intermediate 7 was prepared in a similar manner.

### Intermediate 7

### 3-Bromo-4-(1-piperazinyl)benzonitrile (26.6g) as a yellow solid.

From 3-bromo-4-fluorobenzonitrile (20.0g).
T.l.c. SiO₂ (System A 95:5:0.5), Rf 0.15.

### Intermediate 8

### 4-Cyanophenylboronic acid

A solution of n-butyllithium in hexane (1.58M; 383ml) was added dropwise, at ca. -100° under nitrogen to a stirred solution of 4- bromobenzonitrile (100g) in freshly distilled tetrahydrofuran (1.9L). Addition was complete in 50min and the temperature was maintained at ca. -100° for 15min before triisopropylborate (140ml) was added dropwise between -97° and -96°. Addition was complete in 1h and stirring was continued at ca. -97° for 2.5h. The mixture was allowed to warm to -10° and hydrochloric acid (2M; 352ml) was added dropwise over 10min. The reaction mixture was poured into water (11) and the layers were separated. The organic layer was washed with water (11), saturated brine (11), dried (MgSO₄), filtered and concentrated to give the title compound (60.0g) as a white solid.
N.m.r. (δ, D₆-DMSO): 7.82, 7.96 (4H,AA′BB′, aromatics), 8.45 (2H,br.s, OH).

### Intermediate 9

### 4-(4-Pyridyl)benzonitrile

A mixture of Intermediate 8 (6g), 4-bromopyridine hydrochloride (9.93g), sodium carbonate (18g) and tetrakis(triphenylphosphine)palladium (0) (2.36g) in a mixture of water (70ml) and 1,2-ethanediol, dimethyl ether (140ml) was heated at reflux, under nitrogen, for 20h. The reaction mixture was allowed to cool to room temperature and was concentrated in vacuo. The residue was partitioned between water (300ml) and ethyl acetate (300ml). The aqueous fraction was extracted with ethyl acetate (300ml) and the combined organic fractions were washed with water (300ml) and saturated brine (300ml), dried (MgSO₄), filtered and concentrated in vacuo to give an off-white solid. Purification by dry column 'flash' chromatography using silica gel (Merck 7736) eluting initially with dichloromethane and finally with System A (96:4:0.4) gave the title compound (3.47g) as a colourless solid.
T.l.c. SiO₂ (System A 95:5:0.5), Rf 0.5.

### Intermediate 10

### 4-(4-Cyanophenyl)-1-(phenylmethyl)pyridinium bromide

A mixture of Intermediate 9 (7.62g) and benzyl bromide (5.6ml) in absolute ethanol (200ml) was heated at reflux under nitrogen for 4 days. A further quantity of benzyl bromide (1.4ml) was added and the reaction was heated at reflux for a further 20h. The reaction mixture was allowed to cool to room temperature and the solvent was removed in vacuo. The residue was triturated with a mixture of hexane and ethyl acetate (3:1; 200ml) to give the title compound (12.5g) as a light brown solid.
N.m.r. (δ, D₆-DMSO): 5.9 (2H,s, CH₂Ph), 7.4-7.7 (5H,m, CH₂Ph), 8.16, 8.27, 8.64, 9.38 (8H,2xAA′BB′, aromatics).

### Intermediate 11

### 4-[1-(Phenylmethyl)-4-piperidinyl]benzonitrile

A mixture of Intermediate 10 (12.4g) in a mixture of absolute ethanol (250ml) and dry dimethylformamide (50ml) was cooled to ca. 0° under nitrogen. Sodium borohydride (2.68g) was added portionwise over 10min. The temperature was maintained at ca. 0° for a further 15min. The reaction mixture was allowed to warm to room temperature and stirring was continued under nitrogen for 18h. The solvent was removed in vacuo and the black residue was pre-adsorbed onto silica gel (Merck 7734) and filtered through a column of silica gel (Merck 9385) eluting with System A (98:2:0.2) and evaporating to give a brown oil. This oil was hydrogenated in absolute ethanol (250ml) with 20% palladium hydroxide on carbon (1:1 paste with water; 2g) at room temperature and pressure for 36h. The reaction mixture was filtered through hyflo, and the solvent was removed in vacuo to give a brown oil. Purification by dry column 'flash' chromatography using silica gel (Merck 7736) eluting with hexane:dichloromethane:ethanol:0.880 ammonia (100:50:1:0.1 and 50:50:1:0.1) gave the title compound as a yellow oil (4.5g).
T.l.c. SiO₂ (System A 98:2:0.2), Rf 0.75.

### Intermediate 12

### 4-(4-Piperidinyl)benzonitrile

A solution of Intermediate 11 (1.13g) and 2M aqueous hydrochloric acid (2ml) in absolute ethanol (150ml) was hydrogenated over 20% palladium hydroxide on carbon (1:1 paste with water, 285mg) at room temperature and pressure for 21h. The mixture was filtered through hyflo, the solvent removed in vacuo and the solid residue partitioned between ethyl acetate (100ml) and sodium carbonate (2N; 100ml). The aqueous fraction was extracted with ethyl acetate (100ml) and the combined organic fractions were washed with brine (50ml), dried (MgSO₄), filtered and concentrated in vacuo to give the title compound (547mg) as a colourless oil.
T.l.c. SiO₂ (System A 95:5:0.5), Rf 0.1.

### Intermediate 13

### 1,1-Dimethylethyl 4-[4-(4-cyanophenyl)-1-piperazinyl]-1- hydroxycyclohexaneacetate

Intermediate 6 (1.31g) was heated under reflux with Intermediate 2 (1.59g) and p-toluenesulphonic acid (50mg) in dry toluene (50ml) containing molecular sieves (2g) with stirring under nitrogen for 16h. The mixture was filtered, diluted with ethanol (70ml) and hydrogenated over 5% platinum on carbon (250mg) at room temperature and pressure for 5 days. The catalyst was filtered off and the solvent evaporated to give a pale green oil. Purification by flash chromatography on silica gel (Merck 9385) eluting with System A (96:4:0.4) gave the title compound as a pale yellow oil (1.09g).
T.l.c. SiO₂ (System A 89:10:1),Rf 0.4 (mixture of isomers).

### Intermediate 14

### 1,1-Dimethylethyl 4-[4-(2-bromo-4-cyanophenyl)-1-piperazinyl]-1- hydroxycyclohexaneacetate

A solution of Intermediate 2 (10.0g) in methanol (130ml) was treated with Intermediate 7 (11.7g) and 1M hydrochloric acid (11ml) with stirring under nitrogen. 3 molecular sieves (3.5g) and sodium cyanoborohydride (2.76g) were added and the mixture was stirred at room temperature for 44h. The solvent was evaporated and the residue partitioned between 8% aqueous sodium bicarbonate (250ml) and ethyl acetate (3x100ml). The organic layers were washed with brine, dried (MgSO₄) and evaporated to give a yellow oil (23.0g). Purification by flash chromatography on silica gel (Merck 9385) eluting with System A (95:5:0.5) gave the title compound as a pale yellow oil (9.47g).
T.l.c. SiO₂ (System A 95:5:0.5), Rf 0.3.

### Intermediate 15

### cis-1,1-Dimethylethyl 4-[4-(4-cyanophenyl)-1-piperidinyl]-1-hydroxycyclohexaneacetate

A solution of Intermediate 12 (0.590g) and Intermediate 2 (0.880g) in methanol (20ml) was treated with a solution of hydrogen chloride in ethanol (1M; 0.4ml) and 3 molecular sieves (0.260g). The mixture was stirred at room temperature under nitrogen for 25min. Sodium cyanoborohydride (0.206g) was added in one portion and the reaction mixture was stirred at room temperature under nitrogen for 66h. A further quantity of Intermediate 2 (0.440g) was added in one portion, followed by sodium cyanoborohydride (0.103g), 3 molecular sieves (0.260g) and ethanolic hydrogen chloride (1M; 0.2ml). The reaction was stirred under nitrogen at room temperature, for 48h. The suspension was filtered through hyflo and the residue washed with methanol (40ml). The filtrate and washings were evaporated in vacuo and the residue was partitioned between 8% sodium bicarbonate (50ml) and ethyl acetate (50ml). The aqueous fraction was extracted with ethyl acetate (50ml) and the combined organic fractions were washed with brine (50ml), dried (MgSO₄), filtered and evaporated in vacuo to give a colourless oil. Purification by preparative high performance liquid chromatography (h.p.l.c.; gradient profile: 30-50% (B) in 15min at 45ml/min; R_{T}: 12.83min) gave the title compound (0.19g) as a white solid.
T.l.c. SiO₂ (System A 95:5:0.5), Rf 0.45.

### Intermediate 16

### 1,1-Dimethylethyl 4-[4-[4-(aminothioxomethyl)phenyl]-1-piperazinyl]-1-hydroxycyclohexaneacetate

Intermediate 13 (1.0g) was dissolved in dry dimethylformamide (30ml) and treated with triethylamine (3.7ml). Hydrogen sulphide was bubbled through the mixture for 20 min, and the mixture was stirred at room temperature for 24h and allowed to stand at room temperature for 4½ days. The mixture was poured into 2N aqueous sodium carbonate (150ml) and extracted with ethyl acetate (3x120ml). The organic layers were washed with 50:50 brine: water (4x125ml) and brine, dried (MgSO₄) and evaporated to give the title compound as a pale yellow solid (1.06g).
T.l.c. Si0₂ (System A 89:10:1), Rf 0.5 (mixture of isomers).

Intermediate 17 was prepared in a similar manner.

### Intermediate 17

### 1,1-Dimethylethyl 4-[4-[4-(aminothioxomethyl)-2-bromophenyl]-1-piperazinyl]-1-hydroxycyclohexaneacetate (0.478g) as a yellow gum

From Intermediate 14 (0.5g) in dimethylformamide (15ml) and triethylamine (1.55ml).
T.l.c. SiO₂ (System A 95:5:0.5), Rf 0.17, 0.2 (mixture of isomers ca. 50:50).

### Intermediate 18

### 1.1-Dimethylethyl 4-[4-[2-(aminothioxomethyl)-5-pyrimidinyl]-1-piperazinyl]-1-hydroxycyclohexaneacetate

Intermediate 5 (0.885g) was dissolved in dry dimethylformamide (50ml) and triethylamine (3ml) added. The solution was saturated with gaseous hydrogen sulphide over 0.5h then stirred at 21° for 16h. Ethyl acetate (300ml) was added and the solution washed with 2M sodium carbonate (100ml), then water (3x50ml) and brine (50ml). The organic phase was dried (Na₂SO₄) and evaporated in vacuo. The residue was triturated with hexane-ether (1:1, 50ml) to afford the title compound (0.355g) as a mixture of isomers.
T.l.c. SiO₂ (System A.89:10:1), Rf 0.38, 0.44.

### Intermediate 19

### cis-1,1-Dimethylethyl 4-[4-[4-(aminothioxomethyl)phenyl]-1-piperidinyl]-1-hydroxycyclohexaneacetate

Hydrogen sulphide gas was bubbled through a solution of Intermediate 15 (0.190g) and dry triethylamine (2ml) in dry dimethylformamide (20ml) for 20min. The green solution was stirred at room temperature for 16h. The solvent was evaporated in vacuo and the residue was partitioned between sodium carbonate (2N; 50ml) and methyl ethyl ketone (50ml). The aqueous fraction was extracted with methyl ethyl ketone (50ml) and ethyl acetate (25ml) and the combined organic fractions were washed with brine (100ml), dried (MgSO₄), filtered and concentrated in vacuo to give a brown gum. Trituration with hexane:diethyl ether (3:1; 20ml) gave the title compound (0.208g) as a brown solid.
T.l.c. SiO₂ (System A 95:5:0.5), Rf 0.15.

### Intermediate 20

### cis-1,1-Dimethylethyl 4-[4-[4-(aminoiminomethyl)phenyl]-1-piperazinyl]-1-hydroxycyclohexaneacetate trifluoroacetate

Intermediate 16 (1.0g) was heated under reflux with iodomethane (0.16ml) in acetone (50ml) with stirring under nitrogen for 2.5h. More iodomethane (0.016ml) was added, and heating under reflux was continued for 1h. The solvent was vaporated to give a light brown solid which was dissolved in methanol (50ml) and heated under reflux with ammonium acetate (0.712g) with stirring under nitrogen for 6h. The solvent was evaporated to give a yellow solid. Purification by preparative h.p.l.c. (gradient profile: 10-60% (B)in 17min; R_{T} 13min) gave the title compound as a pale yellow solid (0.335g). N.m.r. (δ,D6-DMSO): 1.43(9H,s,tBu), 1.52, 1.74- 2.04(8H,m,cyclohexane), 2.34(2H,s,CH₂ CO₂tBu),3.08.3.30(5H,m,piperazine + cyclohexane C-H),3.62 (2H,m) & 4.16 (2H,m) (piperazine),7.19(2H) and 7.8 (2H) (aromatics),8.82 9.02 (4H,m,amidine).

### Intermediate 21

### cis-1,1-Dimethylethyl 4-[4-[4-(aminoiminomethyl)-2-bromophenyl]-1-piperazinyl]-1-hydroxycyclohexaneacetate, trifluoroacetate salt

Intermediate 17 (0.470g) was heated under reflux with iodomethane (0.07ml) in acetone (30ml) with stirring under nitrogen for 4h. More iodomethane (0.035ml) was added, and heating under reflux was continued for 3h. The solvent was evaporated and the residue dissolved in methanol (20ml) and heated under reflux with ammonium acetate (0.284g) with stirring under nitrogen for 8h. The solvent was evaporated and the residue purified by preparative h.p.l.c. (gradient profile: 10-70% (B) in 18 min; R_{T} : 13.2min) to give the title compound as a pale yellow oil (0.137g).
Mass spectrometry [MH⁺] 495,497.

### Intermediate 22

### cis-1,1-Dimethylethyl 4-[4-[2-(aminoiminomethyl)-5-pyrimidinyl]-1-piperazinyl]-1-hydroxycyclohexaneacetate, trifluoroacetate salt

Intermediate 18 (0.355g) in acetone (40ml) was treated with iodomethane (70µl) and heated at reflux under nitrogen for 2.5h. Further iodomethane (20µl) was added and the solution heated at reflux for a further 0.5h. The solvent was removed in vacuo, the residue taken up in methanol (30ml), ammonium acetate (0.6g) added and the solution heated at reflux under nitrogen for 3h. The mixture was evaporated in vacuo and the residue purified by preparative h.p.l.c. (isochratic eluent: 35% (B); R_{T} : 10.5min) to afford the title compound as an off-white solid (0.251g).
N.m.r. (δ, D₆-DMSO): 1.36-1.60 (11H, s+m, t-Bu + cyclohexane), 1.72-1.94 (6H, m, cyclohexane) 3.14-3.40 (5H, m, cyclohexane CH + piperazine), 3.63 and 4.37 (4H, 2 x m, piperazine), 4.62 (1H, br. s, OH), 8.75 (2H, s, pyrimidine), 9.26-9.44 (4H, m, amidine), 9.8 (1H, br.s, NH⁺).

### Intermediate 23

### cis-1,1-Dimethylethyl 4-[4-[4-(Aminoiminomethyl)phenyl]-1-piperidinyl]-1-hydroxycyclohexaneacetate

A mixture of Intermediate 19 (0.206g) and iodomethane (78µl) in dry acetone (5ml) was heated at reflux for 2.25h, under nitrogen. The reaction mixture was allowed to cool to room temperature and the solvent was removed in vacuo. The solid residue was heated under reflux with ammonium acetate (0.147g) in methanol (5ml) under nitrogen for 8h. After cooling, evaporation of the solvent in vacuo gave a brown residue which was purified by preparative h.p.l.c. (gradient profile 10-70% (B) in 18min; R_{T}: 12.9min) to give the title compound (0.108g) as a pale yellow solid.
N.m.r (δ, D₆-DMSO): 1.43 (9H,s,^{t}Bu), 1.54, 1.76-2.02, 2.08, 3.58 (14H,m, cyclohexane and piperidine), 2.34 (2H,s,CH₂CO₂^{t}Bu), 3.01 (1H,tt,piperidine CH), 3.06-3.26 (3H,m, cyclohexane CH and piperidine), 7.5, 7.82 (4H,AA′BB′, aromatics), 8.96-9,34 (5H,3xm, amidine and piperidine NH⁺).

### Intermediate 24

### 1,1-Dimethylethyl 4-[4-(2-cyano-5-pyridinyl)-1-piperazinyl]-1-hydroxycyclohexaneacetate

5-Bromo-2-cyanopyridine¹ (1.03g) and Intermediate 4 (1.52g) in dimethylsulphoxide (50ml) were treated with sodium bicarbonate (950mg) and heated at 120° for 16h. The mixture was cooled, poured into water (150ml) and extracted with chloroform (2x220ml, 1x50ml). The combined chloroform extracts were washed with water (200ml), dried (Na₂SO₄) and evaporated in vacuo. The dark residue was purified by flash chromatography over silica (Merck 9385) eluting with dichloromethane-methanol - 0.880 ammonia (95:5:0.5) to afford the title compound as a pale yellow solid (0.755g).
T.l.c. SiO₂ (dichloromethane-methanol - 0.880 ammonia 89:10:1), Rf 0.5.
1. T. Sakamoto, S. Kaneda, S. Nishimura and H. Yamonaka, Chem. Pharm. Bull., 1985, 565.

### Intermediate 25

### 1,1-Dimethylethyl 4-[4-[2-(aminothioxomethyl)-5-pyridinyl]-1-piperazinyl]-1-hydroxycyclohexaneacetate

Intermediate 24 (0.745g) in dimethylformamide (120ml) was treated with triethylamine (5ml) and hydrogen sulphide passed through for 30min. The mixture was allowed to stand for 3 days, then concentrated in vacuo. The residue was poured into 2M sodium carbonate (250ml) and extracted with ethyl acetate (2x200ml). The extracts were washed with water (3x100ml) and brine (100ml), dried (Na₂SO₄) and evaporated in vacuo. The residue was triturated with hexane-ether (1:1,30ml) to afford the title compound as a yellow solid (0.702g).
T.l.c. SiO₂ (triethylamine doped, ethyl acetate-ethanol 19:1), Rf 0.45.

### Intermediate 26

### 1,1-Dimethylethyl 4-[4-[2-[(amino)iminomethyl]-5-pyridinyl]-1-piperazinyl]-1-hydroxycyclohexaneacetate, trifluoroacetate salt

Intermediate 25 (0.694g) in acetone (80ml) was treated with iodomethane (0.15ml) and heated at reflux for 1.5h before being treated with further iodomethane (0.15ml) and heated at reflux for a further 1.5h. The mixture was cooled and evaporated in vacuo and the residue dissolved in methanol (80ml) and ammonium acetate (1g) added. The solution was heated to reflux for 6h and evaporated in vacuo. The residue was purified by preparative h.p.l.c. (gradient profile: 10-70% (B) in 18 min; R_{T}: 11.5-12.0min) to afford the title compound as a colourless solid (0.504g).
Analytical h.p.l.c. retention time 10.7min; gradient profile 10-90% B in 25 min.

### Intermediate 27

### 1,1-Dimethylethyl 4-[4-(5-cyano[1,1′-biphenyl]-2-yl]-1-piperazinyl]-1-hydroxycyclohexaneacetate

Intermediate 14 (0.700g) was heated under reflux with phenylboronic acid (0.214g), tetrakis(triphenylphosphine)palladium (0) (0.089g), sodium carbonate (0.53g) and water (3ml) in 1,2- dimethoxyethane (15ml) with stirring under nitrogen for 16h. The mixture was partitioned between water (40ml) and ethyl acetate (3x25ml) and the organic layers were washed with brine, dried (MgSO₄) and evaporated to give a brown oil. Purification by flash chromatography on silica gel (Merck 9385) eluting with System A (96:3.5:0.35) gave the title compound as a light brown foam (0.647g).
T.l.c. SiO₂ (System A 95:5:0.5), Rf 0.5.

### Intermediate 28

### 1,1-Dimethylethyl 4-[4-(5-(aminothioxomethyl)[1,1′-biphenyl]-2-yl]-1-piperazinyl]-1-hydroxycyclohexaneacetate

Hydrogen sulphide was bubbled through a solution of Intermediate 27 (0.63g) and triethylamine (1.96ml) in dry N,N-dimethylformamide (15ml) at room temperature under nitrogen for 10min. Stirring was continued at room temperature for 21h. The mixture was poured into 2N aqueous sodium carbonate (80ml) and extracted with ethyl acetate (3x60ml). The organic layers were washed with brine-water (50:50; 4x50ml) and brine, dried (MgSO₄) and evaporated to give the title compound as a yellow gum (0.598g).
T.l.c. SiO₂ (System A 95:5:0.5), Rf 0.15, 0.2 (isomers).

### Intermediate 29

### 1,1-Dimethylethyl 4-[4-[5-(aminoiminomethyl)[1,1′-biphenyl]-2-yl]-1-piperazinyl]-1-hydroxycyclohexaneacetate, trifluoroacetate salt

Intermediate 28 (0.588g) was heated under reflux with iodomethane (0.086ml) in acetone (20ml) with stirring under nitrogen for 1.75h. More iodomethane (0.014ml) was added and heating under reflux was continued for 1.5h. The solvent was evaporated to give a yellow foam which was dissolved in methanol (25ml) and heated under reflux with ammonium acetate (0.355g) with stirring under nitrogen for 2h. The solvent was evaporated to give a yellow gum. Purification by preparative h.p.l.c, (gradient profile 10-70% (B) in 18min; R_{T}: 15min) gave the title compound as a pale yellow gum (0.241g).
Mass spectrometry [MH⁺] 493.

### Example 1,

### cis-4-[4-[4-(Aminoiminomethyl)phenyl]-1-piperazinyl]-1-hydroxycyclohexaneacetic acid, trifluoroacetate salt

Intermediate 20 (0.330g) was stirred at room temperature with water (0.5ml) and trifluoroacetic acid (5ml) for 10 min. The solvents were evaporated to give a yellow gum which was triturated with dry ether to give the title compound as a pale yellow solid (0.209g).
N.m.r. (δ, D₆-DMSO): 1.48-2.08 (8H,m,cyclohexane), 2.37 (2H,s,CH₂ CO₂H), 3.08-3.3 (5H,m,piperazine + cyclohexane C-H), 3.62 (2H,dd), 4. 15 (2H,dd)(piperazine), 4.62 (1H,br,OH),7.18(2H),7.8(2H), (aromatics), 8.82, 9.05 (4H,m,amidine).

| | |
|---|---|
| Analysis Found: | C,46.1; H,5.1; N,9.2; |
| C₁₉H₂₈N₄O₃.2CF₃CO₂H.O.5H₂O requires: | C,46.2; H,5.2; N,9.4%. |

### Example 2

### cis-4-[4-[4-(Aminoiminomethyl)-2-bromophenyl]-1-piperazinyl]-1-hydroxycyclohexaneacetic acid, trifluoroacetate salt

Intermediate 21 (0.135g) was dissolved in trifluoroacetic acid (3ml) and water (0.3ml) and the mixture stirred at room temperature for 8min. The solvent was evaporated and the residue purified by preparative h.p.l.c. (gradient profile 10-70% (B) in 18 min; R_{T} : 9.3 min) to give the title compound as a white solid (0.31g)

| | |
|---|---|
| Analysis Found : | C,39.8; H,4.4; N,7.75; |
| C₁₉H₂₇BrN₄O₃.2.5CF₃COOH requires : | C,39.8; H,4.1; N,7.73%. |

N.m.r.(δ, D6-DMSO): 1.44-2.01 (8H, m, cyclohexane), 2.38 (2H, s, CH₂CO₂H), 2.96-3.40 (5H, m, piperazine + cyclohexane CH), 3.56-3.80 (4H, m, piperazine), 4.65 (1H, br. s, OH), 7.42 (1H, d), 7.85 (1H, dd) and 8.14 (1H, d) (aromatics), 9.06 and 9.3 (4H, 2 x br s, amidine), 9.6 and 12.2 (2H, 2 x br s, CO₂H and NH+).

### Example 3

### cis-4-[4-[2-(Aminoiminomethyl)-5-pyrimidinyl]-1-piperazinyl]-1-hydroxycyclohexaneacetic acid, trifluoroacetate salt

Intermediate 22 (0.250g) was dissolved in trifluoroacetic acid (9ml) - water (1ml) and allowed to stand at 22° for 0.5h. The solution was evaporated to dryness in vacuo and the residue triturated with dry ether (2x10ml) to give the title compound as an off-white solid (0.212g).

| | |
|---|---|
| Analysis Found : | C,41.1; H,4.6; N,13.3; |
| C₁₇H₂₆N₆O₃.2.4CF₃COOH requires : | C,41.2; H,4.5; N,13.2%. |

Mass spectrometry [MH⁺] 363.2.

### Example 4

### cis-4-[4-[4-(Aminoiminomethyl)phenyl]-1-piperidinyl]-1- hydroxycyclohexaneacetic acid, trifluoroacetate salt

A solution of Intermediate 23 (0.106g) in a mixture of trifluoroacetic acid (9ml) and water (1ml) was stirred at room temperature under nitrogen for 2h. The solvents were removed in vacuo to give a pale brown residue. Trituration with diethyl ether (20ml), filtration and drying in vacuo gave the title compound (0.058g) as a fawn solid.
Mass spectrometry [MH⁺] 360.
N.m.r. (δ, D₆-DMSO): 1.57, 1.75-2.05, 2.09 (12H,m, cyclohexane and piperidine), 2.37 (2H,s,CH₂CO₂H), 3.01 (1H,tt, piperidine CH), 3.07- 3.28 (3H,m, cyclohexane CH and piperidine), 3.58 (2H,ddd, piperidine), 4.62 (1H,br.s,OH), 7.5, 7.81 (2H,AA′BB′ aromatics), 8.96-9.34 (5H,3xbrm, amidine and piperidine NH⁺).

### Example 5

### cis-4-[4-[2-(Aminoiminomethyl)-5-pyridinyl]-1-piperazinyl]-1- hydroxycyclohexaneacetic acid, trifluoroacetate salt

Intermediate 26 (0.498g) in trifluoroacetic acid-water (9:1, 15ml) was allowed to stand for 2h, then evaporated to dryness in vacuo. The residue was triturated with dry ether (x2) to give the title compound as an off-white solid (0.347g).

| | |
|---|---|
| Analysis Found: | C,42.9; H,4.7; N,10.7; |
| C₁₈H₂₇N₅O₃.2.5CF₃COOH requires | C,42.7; H,4.6; N,10.8%. |

Mass spectrometry [MH⁺] 362.4.

### Example 6

### cis-4-[4-[5-(aminoiminomethyl)[1,1′-biphenyl]-2-yl]-1-piperazinyl]-1-hydroxycyclohexaneacetic acid, trifluoroacetate salt

Intermediate 29 (0.237g) was stirred at room temperature with trifluoroacetic acid (5ml) and water (0.5ml) for 10min. The mixture was evaporated to dryness and the residue triturated with dry ether to give the title compound as a white solid (0.126g).

| | |
|---|---|
| Analysis Found: | C,50.8; H,5.3; N,7.7; |
| C₂₅H₃₂N₄O₃.2.35CF₃COOH requires | C,50.6; H,4.9; N,7.95%. |

N.m.r.(δ, D₆-DMSO): 9.4 (1H,br.s,CO₂H), 9.2 and 8.95 (4H,2xbr.s,NH′s), 7.84 (1H,dd), 7.78-7.70 (3H,m), 7.54 (2H,t), 7.44 (1H,t) and 7.3 (1H,d) (aromatics), 3.1-3.5 (5H,m,N-CH₂′s and N-CH), 2.88-3.02 (4H,m,N-CH₂), 2.35 (2H,s,CH₂CO₂H), 1.9-1.64 (6H,m,cyclohexyl CH₂′s), 1.50 (2H,br.t,cyclohexyl CH₂).

### Example 7

### Biological Data

Inhibition of blood platelet aggregation by compounds of the invention was determined according to the following procedure. Citrated whole blood (1 part 3.8% trisodium citrate: 9 parts blood) was obtained from human volunteers, free of medication for at least 10 days prior to collection. The blood was incubated with 0.1mM aspirin and 0.05µM prostacyclin and then centrifuged at 1000g for 4 minutes (20°C). The supernatant platelet rich plasma (PRP) was further centrifuged at 1300g for 10 minutes (20°C) to sediment the platelets. The supernatant was discarded and the pellet washed with a physiological salt solution (HEPES 5mM, NaHCO₃ 12mM, NaCl 140mM, KH₂PO₄ 0.74mM, D-Glucose 5.6mM, KCl 2.82mM and BSA 20g/l, pH 7.4) to remove residual plasma. After washing, the pellet was resuspended in physiological salt solution and then applied to a sepharose CL-2B column, pre-equilibrated with physiological salt solution at room temperature. The platelets (GFP) eluted within the void volume and were diluted to approximately 300,000 platelets/µl in buffer. Purified human fibrinogen (Knight L.C. et al, 1981 Thromb. Haemostasis, 46(3), 593-596) was added to a final concentration of 0.5mg/ml together with Ca2+ and Mg2+ at 1mM and 0.5mM respectively. Test compounds were incubated in GFP for 5 minutes at 37°C and the platelet aggregating agent adenosine diphosphate (ADP) was added to a final concentration of 1 x 10⁻⁵M. The potency of the compounds may be expressed as an IC₅₀ value, defined as the concentration of compound required to produce 50% inhibition of platelet aggregation.

The following IC₅₀ values were obtained for the compounds of the invention:

| Compound Example No. | IC₅₀ (nM) |
|---|---|
| 1 | 45 |
| 2 | 945 |
| 3 | 543 |
| 4 | 13 |
| 5 | 220 |
| 6 | 1549 |

### Example 8

### Pharmacy Example - Tablets

| | |
|---|---|
| a) Compound of the invention | 5.0mg |
| Lactose | 95.0mg |
| Microcrystalline Cellulose | 90.0mg |
| Cross-linked polyvinylpyrrolidone | 8.0mg |
| Magnesium Stearate | 2.0mg |
| Compression weight | 200.0mg |

The compound of the invention, microcrystalline cellulose, lactose and cross-linked polyvinylpyrrolidone are sieved through a 500 micron sieve and blended in a suitable mixer. The magnesium stearate is sieved through a 250 micron sieve and blended with the active blend. The blend is compressed into tablets using suitable punches.

| | |
|---|---|
| b) Compound of the invention | 5.0mg |
| Lactose | 165.0mg |
| Pregelatinised Starch | 20.0mg |
| Cross-linked polyvinylpyrrolidone | 8.0mg |
| Magnesium Stearate | 2.0mg |
| Compression weight | 200.0mg |

The compound of the invention, lactose and pregelatinised starch are blended together and granulated with water. The wet mass is dried and milled. The magnesium stearate and cross-linked polyvinylpyrrolidone are screened through a 250 micron sieve and blended with the granule. The resultant blend is compressed using suitable tablet punches.

### Example 9

### Pharmacy Example - Capsules

| | |
|---|---|
| a) Compound of the invention | 5.0mg |
| Pregelatinised Starch | 193.0mg |
| Magnesium Stearate | 2.0mg |
| Fill weight | 200.0mg |

The compound of the invention and pregelatinised starch are screened through a 500 micron mesh sieve, blended together and lubricated with magnesium stearate, (meshed through a 250 micron sieve). The blend is filled into hard gelatine capsules of a suitable size.

| | |
|---|---|
| b) Compound of the invention | 5.0mg |
| Lactose | 177.0mg |
| Polyvinylpyrrolidone | 8.0mg |
| Cross-linked polyvinylpyrrolidone | 8.0mg |
| Magnesium Stearate | 2.0mg |
| Fill weight | 200.0mg |

The compound of the invention and lactose are blended together and granulated with a solution of polyvinylpyrrolidone. The wet mass is dried and milled. The magnesium stearate and cross-linked polyvinylpyrrolidone are screened through a 250 micron sieve and blended with the granules. The resultant blend is filled into hard gelatine capsules of a suitable size.

### Example 10

### Pharmacy Example - Syrup

| | |
|---|---|
| a) Compound of the invention | 5.0mg |
| Hydroxypropyl Methylcellulose | 45.0mg |
| Propyl Hydroxybenzoate | 1.5mg |
| Butyl Hydroxybenzoate | 0.75mg |
| Saccharin Sodium | 5.0mg |
| Sorbitol Solution | 1.0ml |
| Suitable Buffers | qs |
| Suitable flavours | qs |
| Purified Water to | 10.ml |

The hydroxypropyl methylcellulose is dispersed in a portion of hot purified water together with the hydroxybenzoates and the solution is allowed to cool to room temperature. The saccharin sodium flavours and sorbitol solution are added to the bulk solution. The compound of the invention is dissolved in a portion of the remaining water and added to the bulk solution. Suitable buffers may be added to control the pH in the region of maximum stability. The solution is made up to volume, filtered and filled into suitable containers.

### Example 11

### Pharmacy Example - Injection Formulation

| | |
|---|---|
| | % w/v |
| Compound of the invention | 1.00 |
| Water for injections B.P. to | 100.00 |

Sodium chloride may be added to adjust the tonicity of the solution and the pH may be adjusted to that of maximum stability and/or to facilitate solution of the compound of the invention using dilute acid or alkali or by the addition of suitable buffer salts. Antioxidants and metal chelating salts may also be included.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

## Claims

1. Compounds of formula (I) 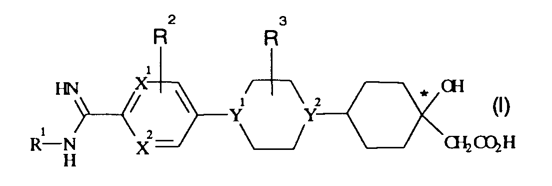 in which
X¹, X² and Y¹, which may be the same or different, represent CH or N;
Y² represents N or, when Y¹ represents N, may also represent CH;
R¹ represents a hydrogen atom or a hydroxyl, C₁₋₄alkyl or 2,2,2- trifluoroethyl group;
R² represents a hydrogen atom or, when both X¹ and X² represent CH, may also represents a fluorine, chlorine or bromine atom or a phenyl or C₁₋₄alkyl group;
R³ represents a hydrogen atom or, when both Y¹ and Y² represent N, may also represents a C₁₋₄alkyl or hydroxymethyl group and salts and solvates thereof.

2. Compounds as claimed in Claim 1 where the compound of formula (I) is a compound of formula (Ia): 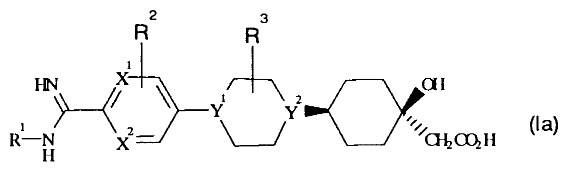 X¹, X² and Y¹, which may be the same or different, represent CH or N;
Y² represents N or, when Y¹ represents N, may also represent CH;
R¹ represents a hydrogen atom or a hydroxyl, C₁₋₄alkyl or 2,2,2- trifluoroethyl group;
R² represents a hydrogen atom or, when both X¹ and X² represent CH, may also represents a fluorine, chlorine or bromine atom or a phenyl or C₁₋₄alkyl group;
R³ represents a hydrogen atom or, when both Y¹ and Y² represent N, may also represents a C₁₋₄alkyl or hydroxymethyl group.

3. Compounds as claimed in Claim 1 or 2 in which one or both X¹ and X² represents CH.

4. Compounds as claimed in any one of Claims 1 to 3 in which one or both Y¹ and Y² represents N.

5. Compounds as claimed in any one of Claims 1 to 4 in which R¹ represents a hydrogen atom.

6. Compounds as claimed in any one of Claims 1 to 5 in which R³ represents a hydrogen atom.

7. cis-4-[4-[4-(Aminoiminomethyl)phenyl]-1-piperazinyl]-1- hydroxycyclohexaneacetic acid;
cis-4-[4-[4-(aminoiminomethyl)-2-bromophenyl]-1-piperazinyl]-1- hydroxycyclohexaneacetic acid;
cis-4-[4-[2-(aminoiminomethyl)-5-pyrimidinyl]-1-piperazinyl]-1- hydroxycyclohexaneacetic acid;
cis-4-[4-[2-(aminoiminomethyl)-5-pyridinyl]-1-piperazinyl]-1-hydroxycyclohexaneacetic acid;
cis-4-[4-[4-(aminoiminomethyl)phenyl]-1-piperidinyl]-1-hydroxycyclohexaneacetic acid;
cis-4-[4-[5-(aminoiminomethyl)[1,1′-biphenyl]-2-yl]-1-piperazinyl]-hydroxycyclohexaneacetic acid; and physiologically acceptable salts and solvates thereof.

8. Compounds as claimed in any of Claims 1 to 7 wherein the compound of formula (I) is in the form of a hydrochloride, hydrobromide, sulphate, phosphate, benzoate, naphthoate, hydroxynaphthoate, p- toluenesulphonate, methanesulphonate, sulphamate, ascorbate, tartrate, salicylate, succinate, lactate, glutarate, glutaconate, acetate, tricarballylate, citrate, fumarate, maleate or sodium salt.

9. A process for the preparation of compounds of formula (I) as defined in any of Claims 1 to 8 or a physiologically acceptable salt or solvate thereof, which comprises:
(A) for the preparation of compounds of formula (I) in which R¹ represents a hydrogen atom alkylating a compound of formula (II) 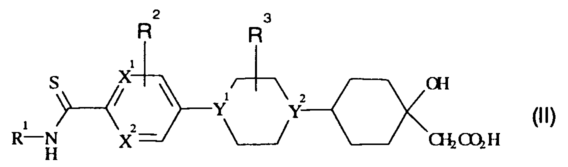 followed by reaction with a source of ammonia and thereafter removing the carboxylic acid protecting group; or
(B) for the preparation of a compound of formula (I) in which R¹ represents a hydroxyl group, treating a compound of formula (III) 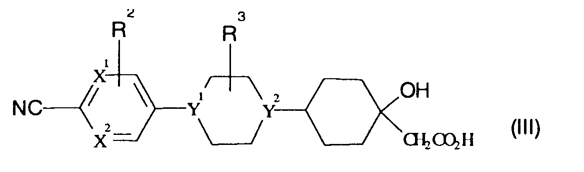 or a protected derivative thereof with hydroxylamine or an acid addition salt thereof in the presence of a base, followed by removing any protecting groups present; or
(C) for the preparation of a compound of formula (I) in which R¹ represents a C₁₋₄alkyl or 2,2,2-trifluoroethyl group, treating a compound of formula (IV) 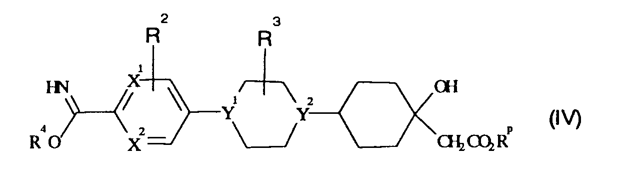 (wherein R⁴ represents an alkyl group) with an amine R¹NH₂ (wherein R¹ represents C₁₋₄alkyl or 2,2,2-trifluoroethyl), followed by removal of the carboxylic acid protecting group; or
(D) deprotecting a protected derivative of a compound of formula (I); or
(E) interconversion of a compound of formula (I) into another compound of formula (I); and when the compound of formula (I) is obtained as a mixture of enantiomers, optionally resolving the mixture to obtain the desired enantiomer, and/or where the compound of formula (I) is in the form of a free base, optionally converting the free base into a salt.

10. A pharmaceutical composition comprising a compound of formula (I) as defined in any one of Claims 1 to 8 or a physiologically acceptable salt or solvate thereof together with at least one physiologically acceptable carrier or excipient.

11. A compound of formula (I) as defined in any of Claims 1 to 8 or a physiologically acceptable salt or solvate thereof for use in human or veterinary medicine.

12. The use of a compound of formula (I) or a physiologically acceptable salt or solvate thereof as defined in any of Claims 1 to 8 for the manufacture of a therapeutic agent for the treatment or phrophylaxis of thrombotic disorders.

13. A method of treating a human or animal subject suffering from or susceptible to a thrombotic disorder, which method comprises administering to said subject an effective amount of a compound of formula (I) as defined in any of claims 1 to 8 or a physiologically acceptable salt or solvate thereof.
